# EUROPÄISCHE PATENTSCHRIFT

(11) **EP 1 315 749 B1**
(45) Veröffentlichungstag und Bekanntmachung des Hinweises auf die Patenterteilung: **19.09.2012**
(21) Anmeldenummer: 01978281.2
(22) Anmeldetag: 18.08.2001
(51) Int. Cl.: C07K 14/47, C07D 205/08, A61K 47/48, A61K 38/17, G01N 33/50

(54) **PROTEIN AUS DEM DARM VON WIRBELTIEREN, WELCHES CHOLESTERIN ABSORBIERT, SOWIE VERWENDUNG DIESES PROTEINS ZUR IDENTIFIZIERUNG VON INHIBITOREN DES INTESTINALEN CHOLESTERINTRANSPORTS**
PROTEIN EXTRACTED FROM THE INTESTINES OF VERTEBRATES, WHICH ABSORBS CHOLESTEROL, AND THE USE OF THIS PROTEIN FOR IDENTIFYING INHIBITORS OF INTESTINAL CHOLESTEROL TRANSPORT
PROTEINE ISSUE DE L'INTESTIN D'ANIMAUX VERTEBRES, QUI ABSORBE LE CHOLESTEROL, UTILISATION DE LADITE PROTEINE POUR IDENTIFIER DES INHIBITEURS DU TRANSPORT INTESTINAL DU CHOLESTEROL

(30) Priorität: 29.08.2000 DE 10042447
(43) Veröffentlichungstag der Anmeldung: 04.06.2003
(73) Patentinhaber: Sanofi-Aventis Deutschland GmbH, 65929 Frankfurt am Main (DE)
(72) Erfinder: KRAMER, Werner, 55130 Mainz-Laubenheim (DE); GLOMBIK, Heiner, 65719 Hofheim (DE)
(86) Internationale Anmeldenummer: PCT/EP2001/009554
(87) Internationale Veröffentlichungsnummer: WO 2002/018432

(56) Entgegenhaltungen:
- WO-A-00/63703
- DETMERS P A ET AL: "A target for cholesterol absorption inhibitors in the enterocyte brush border membrane" BIOCHIMICA ET BIOPHYSICA ACTA, Bd. 1486, Nr. 2-3, 19. Juli 2000 (2000-07-19), Seiten 243-252, XP004277380
- HAUSER H ET AL: "Identification of a receptor mediating absorption of dietary cholesterol in the intestine" BIOCHEMISTRY, AMERICAN CHEMICAL SOCIETY. EASTON, PA, US, Bd. 37, 1998, Seiten 17843-17850, XP002133825 ISSN: 0006-2960
- HERNANDEZ M ET AL: "Intestinal absorption of cholesterol is mediated by a saturable, inhibitable transporter" BIOCHIMICA ET BIOPHYSICA ACTA, Bd. 1486, Nr. 2-3, 19. Juli 2000 (2000-07-19), Seiten 232-242, XP004277379
- ACTON S L ET AL: "EXPRESSION CLONING OF SR-BI, A CD36-RELATED CLASS B SCAVENGER RECEPTOR" JOURNAL OF BIOLOGICAL CHEMISTRY, AMERICAN SOCIETY OF BIOLOGICAL CHEMISTS, BALTIMORE, MD, US, Bd. 269, Nr. 33, 19. August 1994 (1994-08-19), Seiten 21003-21009, XP000644467 ISSN: 0021-9258
- KRAMER ET AL: J. BIOL. CHEM., vol. 280, no. 2, 19 October 2004 (2004-10-19), pages 1306-1320,

## Beschreibung

Die Erfindung betrifft ein Verfahren zur Isolierung eines Proteins aus dem Darm von Wirbeltieren, welches Cholesterin absorbiert. Das Protein wird mittels hochaffiner quervernetzender Verbindungen charakterisiert.

Beim Menschen liegen durchschnittlich etwa 50 % des Cholesterins im Darmlumen vor. Das intraluminale Cholesterin stammt vorwlegend aus der Nahrung sowie der Gallenflüssigkeit. Aus der Gattenflüssigkeit wird pro Tag ca. 2 g Cholesterin abgesondert. Die intestinale Cholesterinabsorption hängt stark vom Vorliegen von Gallensalzen ab. So bewirkt eine Verabreichung von inhibitoren der Gallensalzwiederaufnahme bzw. von Gallensalz-Sequestriermitteln eine Inhibierung der intestinalen Cholesterinabsorption.

Bei der Behandlung von Lipidstörungen, Atherosklerose und Herzkreislauferkrankungen ist die Inhibierung der intestinalen Cholesterinabsorption ein wichtiges Ziel. Nach vorherrschende Meinung in der Fachwelt erfolgt die intestinale Cholesterinabsorption durch physikochemische Diffusion.

Bekannt sind eine Reihe von Beobachtungen im Zusammenhang mit dem Cholesterintransport, die für die Beteiligung eines Proteins sprechen. Die intestinale Cholesterinabsorption unterliegt einer großen individuellen Variabilität. Biochemische Daten aus in vitro Versuchen sprechen für eine Beteiligung von Proteinen beim Cholesterinaustausch zwischen kleinen unilamellaren Vesikeln und den Bürstensaumvesikeln des Darms. Bei der intestinalen Absorption von Pflanzensterinen wie dem β-Sitosterin und Campesterin, die sich lediglich hinsichtlich einer Methylgruppe (β-Sitosterin) bzw. einer Ethylgruppe (Campesterin) unterscheiden, konnten große Unterschiede beobachtet werden. Unter anderem beim Menschen zeigte β-Sitosterin eine Inhibierung der Cholesterinabsorption. Es gibt zwei hochwirksame Verbindungsklassen, welche die Intestinale Cholesterinabsorption inhibieren, wenn eine luminale Verabreichung erfolgt. Die Verbindungen sind zum einen von Saponin abgeleitete Verbindungen wie Tiquesid und Pamaquesid, zum anderen bestimmte Derivate von 2-Azetidinonen.

Derivate von 2-Azetidinonen als Inhibitoren der Cholesterin Absorption sind beschrieben in Clader et al., J. Med. Chem. 38, 3684 - 3693, 1996. Absorption im Sinne dieser Erfindung soll Anlagerung eines Stoffes an ein Protein und Transport diese Stoffes mit Hilfe dieses Proteins bedeuten.

Detmers et al, Biochimica et Biophysica Acta 1486, p. 243-252 (2000) offenbaren die Aufnahme von ³H- markierten Inhibitoren wie z.B. L-166143 durch Zellen aus dem Bürstensaum des Darmgewebes.

Es wurden bislang mehrere Proteine in einen Zusammenhang mit der Cholesterinabsorption gebracht. Es wurde jedoch noch kein Protein beschrieben, welches eindeutig an der Cholesterinabsorption im Darm beteiligt ist. Dies führt zu einer Reihe von Nachteilen, bei der Suche nach einem rationellen Vorgehen bei der identifizierung von spezifischen Inhibitoren der intestinalen Cholesterinabsorption. Das ist besonders auch deshalb von großer Bedeutung, da sich Inhibitoren der Intestinalen Cholesterinabsorption in besonderer Weise zur Behandlung aller Arten von Lipidstörungen, Atherosklerose und Herzkreislauferkrankungen eignen. Cholesterin ist in allen Zellen sowohl in der Lipid-Doppelschicht der Membranen als auch in Lipidvesikel gleichmäßig verteilt. Transportmessungen mit Vesikeln oder Zellen sind deshalb sehr umständlich und störungsanfällig.

In Kramer et al, J. Biol. Chem., 280, p.1306-1320 2005, wurde das Ezetimibe-bindende Protein mit einem Molekulargewicht von 145 kDa als Aminopeptidase N (CD13) identifiziert.

Aufgabe der vorliegenden Erfindung ist deshalb die Bereitstellung eines Verfahrens zur Isolierung eines Proteins, welches an der intestinalen Cholesterinabsorption beteiligt ist.

Die Erfindung betrifft deshalb ein Verfahren zur Isolierung eines Cholesterin absorbierenden Proteins aus dem Darmgewebe eines Säuger-Organismus, wobei das Verfahren die folgenden Verfahrensschritte umfasst:
a) Bereitstellung von Zellen aus dem Bürstensaum des Darmgewebes oder Teilen von diesen Darmzellen eines Säuger-Organismus,
b) Bereitstellung einer radioaktiv markierten Verbindung, welche als Inhibitor der intestinalen Cholesterinabsorption wirkt, und eine photolabile Gruppe enthält,
c) In-Kontakt-Bringen der Darmzellen oder Teilen von diesen Darmzellen aus a) mit einer Verbindung aus b),
d) Bestrahlung des Ansatzes gemäss c) mit UV-Licht,
e) Aufschluss der Zellen nach Bestrahlung gemäss d),
f) Auftrennung der Bestandteile des Aufschlusses der Zellen nach Aufschluss gemäss e),
g) Nachweis eines Proteins nach Auftrennung gemäss f), welches eine Verbindung gemäss b) kovalent gebunden enthält,
dadurch charakterisiert, dass das Protein eine Größe von 145 kDa +/- 7.55 kDa bei der SDS-Polyacrylamid-Gelelektrophorese hat, dass das Protein ein integrales Membranprotein ist und dass das Protein in glykosyliertem Zustand vorliegt,
wobei die Verbindung gemäss Verfahrenschritt b) eine Verbindung mit folgender Formel I 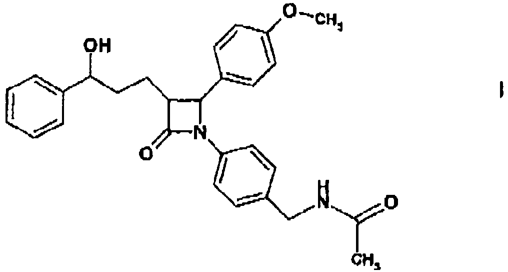 ist oder eine Verbindung mit folgender Formel II 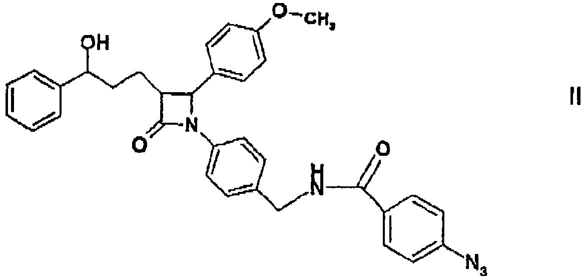 ist.

Die Bereitstellung von Darmzellen kann beispielsweise durch Präparation des Darms aus Tieren und anschließende Reinigung, enzymatischen Aufschluß des Bindegewebes und Suspendierung von Einzelzellen in isotonischen Pufferlösungen erfolgen. Als Darmgewebe zur Bereitstellung von Darmzellen eignen sich unter anderem die übrig gebliebenen entsprechenden Teile von Tieren nach Schlachtungen. Darmzellen können auch aus menschlichem Darmgewebe bereitgestellt werden, nachdem Anteile des Darms operativ gewonnen wurden. Die Darmzellen können auch aus Darmzellkulturen bestehen, die für den Zweck der Erfindung durch Anwendung von Zellkulturtechniken bereitgestellt werden. Teile von Darmzellen können Organellen der Darmzellen sein. Organellen sind inbesondere Membranen der Darmzellen. Membranen der Darmzellen können nach Aufschluß der Zellen durch differentielle Zentrifugation gewonnen werden. Teile von Darmzellen sind insbesondere auch Proteinfraktionen. Bereitstellung von Darmzellen oder Teilen von Darmzellen sind dem Fachmann, in diesem Fall einem Biochemiker, geläufig. insbesondere wird auf folgende Lehrbücher verwiesen: "Basic Cell Culture, A practical approach, IRI. press (1994), Herausgeber: J.M. Davis" sowie auf "Current Protocols in Protein Science, John Wiley & Sons (2000), Herausgeber: J. E. Coligan, B. M. Dunn, H. L. Ploegh, D. W. Speicher, P. T. Wingfield".

In einer bevorzugten Ausführungsform der Erfindung werden Darmzellen von Mensch, Affe, Rind, Schwein, Ratte, Maus, Kaninchen oder Hamster verwendet.

Als Verbindung, welche als inhibitor der intestinalen Cholesterinabsorption wirkt und die eine photolabile Gruppe enthält, wird folgende Verbindung der Formel I verwendet, welche radioaktiv markiert ist.

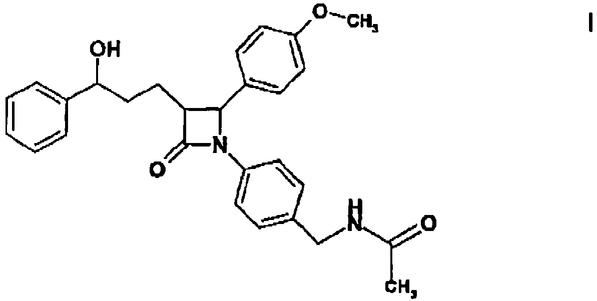

Die Verbindung der Formel I hat die Bezeichnung N-{4-[3-(3-hydroxy-3-phenylpropyl)-2-(4-methoxy-phenyl)-4-oxo-azetidin-1-yl]-benzyl}-acetamid.

Als Verbindung, welche als Inhibitor der intestinalen Cholesterinabsorption wirkt, und die eine photolabile Gruppe enthält, wird auch folgende Verbindung der Formel II verwendet, welche radioaktiv markiert ist.

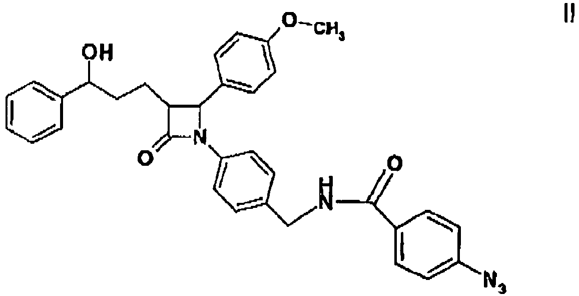

Die Verbindung der Formel II hat die Bezeichnung 4-Azido-N-{4-[3-(3-hydroxy-3-phenyl-propyl)-2-(4-methoxy-phenyl)-oxo-azetidin-1-yl]-benzyl}-benzamid.

Die Beschreibung der Synthese einer Verbindung der Formel I und II erfolgt in einem nachfolgenden Absatz in den Beispielen.

Das Protein, das gemäß einen Verfahren der Erfindung besgestellt wird, kann bevorzugt einen inhibitor der intestinalen Cholesterinabsorption binden. Besonders bevorzugt bindet das Protein Azetidinone oder Saponine, Weiterhin bevorzugt kann das Protein Cholesterin binden. Die Bestimmung der Inhibition der intestinalen Cholesterinabsorption erfolgt beispielsweise mit isolierten menschlichen oder tierischen Darmzellen, Darmzellinien oder Teilen eines menschlichen oder tierischen Darms. Hierzu wird mit markiertem Cholesterin die Absorption an die genannten Zellen bestimmt. Die Markierung des Cholesterin kann aus einer radioaktiven oder anderen Markierung bestehen, Als radioaktive Markierung können ³H, ¹⁴C oder anderen Isotope verwendet werden, die mittels dem Fachmann bekannten synthetischen Methoden in das Molekülgerüst des Cholesterin eingesetzt werden. Ein Inhibitor der intestinalen Cholesterinabsarption vermindert die Menge an Cholesterin, die von den Darmzellen absorbiert wird.

Das Protein hat eine Größe von 145 kDa +/- 7,55 kDa bei der SDS-Polyacrylamid-Gelelektrophorese. Die Bestimmung der Größe des Proteins kann beispielsweise durch Anwendung von denaturierender Polyacrylamidelektrophorese im Vergleich mit Größenmarkern, durch Hochdruckflüssigkeitschromatographie im Vergleich mit Größernmarkern. Gelchromatographie im Vergleich mit Größenmarkern, Massenspektrometrie oder anderen Methoden erfolgen. Weiter hin ist das Protein glykosyliert. Die glykosylierte Form eines Proteins erkennt man daran, daß sich die Größe des Proteins vermindert, wenn das Protein mit einer Glykosidase behandelt wird. Angaben zu geeigneten Methoden zur Überprüfung der Glykosylierung findet der Fachmann In "Carbohydrate Biotechnology Protocols, Methods in Biotechnology, 10 (1999) Humana Press, ISBN 0-89603-563-8, Herausgeber C. Bucke".

Das In-Kontakt-Bringen der Darmzellen oder von Teilen aus diesen Darmzellen Insbesondere von Membranen mit der radioaktiv markierten Verbindung enthaltend einen inhibitor der intestinalen Cholesterinabsorption und eine photolabile Gruppe, kann in gewöhnlichen Laborbehältnissen wie beispielsweise Eppendorf-Gefäßen, Zentrifugen-Röhrchen oder Glaskolben erfolgen. Das zugrunde liegende Medium enthält beispielsweise Puffersubstanzen, Nährmediumbestandteile, Salze, Spurenelemente und anderes in wässriger Lösung.

Eine photolabile Gruppe in einem Molekül kann dazu verwendet werden, kovalente Bindungen zu einem in unmittelbarer Nähe befindlichen Molekül insbesondere einem Protein herzustellen. Dazu wird die Verbindung mit der photolabilen Gruppe zuerst in unmittelbare Nähe des Moleküls gebracht, zu dem die kovalente Bindung hergestellt werden soll. Dies kann beispielsweise durch einen anderen Teil geschehen, der als spezifischer inhibitor - im vorliegenden Fall der Cholesterinabsorption - eines Proteins funktioniert. Nach In-Kontakt-Bringen der Moleküle wird mit UV-Licht bestrahlt. Die Bestrahlung mit UV-Licht aktiviert die photolabile Gruppe und initiiert die Herstellung einer kovalenten Bindung mit dem wechselwirkenden Molekül insbesondere mit einem Protein. Als photolabile Gruppe eignen sich beispielsweise Diazirin, Azido oder Carbonylfunktionen,

Zur Bestrahlung kann eine gebräuchliche UV-Lampe wie sie beispielsweise zum Sichtbarmachen von Polynukleotiden mit eingelagertem Ethidiumbromid oder zur Entkeimung von Laborflächen verwendet wird oder ein photochemischer Reaktor (erhältlich unter anderem von der Firma "The Southern Ultraviolet Company, Hamden, CT") eingesetzt werden. Der Aufschluß der Zellen nach Bestrahlung mit UV-Licht wird mit üblicherweise zum Zellaufschluß angewendeten Methoden durchgeführt. Solche sind beispielsweise wiederholtes Einfrieren und Wiederauftauen, die Behandlung der Zellen mit Ultraschall, die Verwendung einer French-Press oder die Zugabe eines Detergens und Enzymen, Die Auftrennung der Proteine des Zelllysates kann beispielsweise durch Fällung mit Ammonsulfat, durch differentielle Zentrifugation oder Anwendung von chromatographischen Techniken ausgeführt werden. Hierfür geeignete chromatographische Techniken sind beispielsweise denaturierende oder nichtdenaturierende Polyacrylamidelektrophorese in einer oder zwei Dimensionen, die Hochdruckflüssigkeitschromatographie, **I**onenaustauschchromatographie oder Affinitätschromatographie. Diese Techniken sind dem Fachmann geläufig und werden beispielsweise ausführlich in den bereits erwähnten "Current Protocols In Protein Science" abgehandelt.

Der Nachweis eines Proteins nach der Auftrennung erfolgt mittels der radioaktiven Markierung der Verbindung enthaltend einem Inhibitor der Intestinalen Cholesterinabsorption und eine photolabilen Gruppe. Als radioaktives Isotop hierfür kann beispielsweise ³H oder ¹⁴C verwendet werden. Als Nachweismethode eignet sich beispielsweise die Erfassung des Proteins, welches eine Verbindung kovalent gebunden enthält, mittels eines zur Röntgenphotographie verwendeten Filmmaterials, nachdem das Protein mit Hilfe einer Polyacrylamidgelelektrophorese auf einem Polyacrylamidgel aufgebracht wurde. Andere geeignete Nachweismethoden sind Flüssigkeits-Szintillations-Zählung oder Flachbett-Scanning.

Zur Bereitstellung eines Proteins gemäβ dem Verfahren der Erfindung können grundsätzlich alle Zellen, welche solch ein Protein ausbilden verwendet werden. Im Rahmen der Erfindung werden Zellen aus dem Burstensaum des Darmgewebes von Säuger-Organismen verwendet. Als Säuger-Organismen, aus denen diese Darmzellen gewonnen werden, können beispielsweise Mensch, Affe, Rind, Schwein, Ratte, Maus, Kaninchen, Hamster oder eine andere Wirbeltierspezies ausgewählt werden. Die Bereitstellung kann durch Präparation von Zellsuspensionen aus dem Bürstensaumgewebe des Darms solcher Organismen erfolgen. Geeignetes Material des Darms erhält man beispielsweise durch operative Eingriffe. Andere Quellen können sich aus übrig gebliebenen Teilen von Tieren nach Schlachtungen ergeben. Ebenso geeignet sind Zellen einer Darmzellinie. Das Darmgewebe kann zur Herstellung geeigneter zellpräparationen einer Enzymbehandlung zur Freisetzung von Einzelzellen unterworfen und anschließend differentiell zentrifugiert werden. Die entstehenden Zellen oder Organellen werden anschließend In geeigneten wäßrigen Medien aufgenommen. Diese wäßrige Medien können Puffersubstanzen, Salze, Proteine sowie weiterhin Hilfsstoffe enthalten.

Die Erfindung beschreibt ein Verfahren zur Gewinnung eines Proteins der Erfindung wobei das Verfahren folgende Verfahrensschritte enthält:
a) Bereitstellung einer Zelle enthaltend ein Protein der Erfindung.
b) Bereitstellung eines chemischen Konjugats bestehend aus einem Polymer, wobei das Polymer eventuell über einen Spacer an einen Inhibitor der intestinalen Cholesterinabsorption kovalent gebunden ist,
c) Aufschluß der Zelle aus a)
d) in-Kontakt-Bringen des Aufschlusses aus c) mit einem chemischen Konjugat aus b),
e) Separierung der nicht gebundenen Proteine und anderen Moleküle des Aufschlussesaus c) vom chemischen Konjugat,
f) Elution der Proteine, welche nach In-Kontak-Bringen gemäß d), mit chemischen Konjugat eine stabile Verbindung eingegangen sind sowie gegebenenfalls weitere Reinigung.

Als Zelle, welche ein Protein dieser Erfindung enthält, kann eine Darmzelle eines Wirbeltiers verwendet werden. Als Säuger-Organismen, aus denen solche Darmzellen gewonnen werden, können beispielsweise Mensch, Affe, Rind, Schwein, Ratte, Maus, Kaninchen oder Hamster ausgewählt werden. Die Bereitstellung kann durch Präparation von Zellsuspensionen aus dem Bürstensaumgewebe des Darms erfolgen. Geeignetes Material des Darms erhält man beispielsweise durch operative Eingriffe. Andere Quellen können sich aus übrig gebliebenen Teilen von Tieren nach Schlachtungen ergeben. Geeignet sind auch Zellen einer Darmzellinle. Das Darmgewebe wird zur Freisetzung von Einzelzellen beispielsweise einer Enzymbehandlung unterworfen oder differentiell zentrifugiert. Die entstehenden Präparationen der Zellen oder Organellen werden anschließend in geeigneten wäßrigen Medien aufgenommen. Diese wäßrige Medien kannen Puffersubstanzen. Salze, Proteine sowie weiterhin Hilfsstoffe enthalten. Die Zellpräparation wird zur Gewinnung eines Proteins dieser Erfindung einem Aufschluß unterworfen. Der Aufschluß der Zellen wird mit den üblicherweise zum Zellaufschluß angewendeten Methoden durchgeführt. Solche sind beispielsweise wiederholtes Einfrieren und Wiederauftauen, die Behandlung der Zellen mit Ultraschall, die Verwendung einer French-Press oder die Zugabe eines Detergens und Enzymen.

Die Bereitstellung eines chemischen Konjugat bestehend aus einem Polymer, wobei das Polymer eventuell über einen Spacer an einen Inhibitor der Intestinalen Cholesterinabsorption kovalent gebunden ist, erfolgt durch chemische Synthese. Das Konjugat wird nach der Synthese in ein wäßriges oder organisches Lösungsmittel aufgenommen. Die stabile Verbindung eines Proteins zu diesem chemischen Konjugat kann über affine Bindungen, über hydrophile oder hydrophobe Wechselwirkung oder in anderer Weise erfolgen. Diese Verbindung des Proteins mit dem chemischen Konjugat kann durch Zuführen von geeigneten Elutionsmitteln wieder gelöst werden. Solche geeigneten Elutionsmittel enthalten beispielsweise hohe Konzentrationen eines Stoffes, der sich zu einer affinen oder anderen Wechselwirkung kompetitiv verhält.

Der Aufschluß der Zellen, der wie soeben beschrieben hergestellt wurde, wird mit dem chemischen Konjugat in Kontakt gebracht. Dazu kann das chemische Konjugat zuvor als Suspension in eine Chromatographlesäule eingefüllt werden. Der Aufschluß wird dann nach Packen der Säule auf das affinitätschromatographische Material aufgetragen und mit einem wäßrigen Lösungsmittel eluiert, welches Puffersubstanzen, Salze, Proteine und Hilfsstoffe insbesondere Stabilisatoren, Lösungsvermittler oder Konservierungsstoffe enthalten kann. Die Elution bewirkt eine Separierung der nicht gebundenen Proteine und anderer Moleküle, die nicht einem Protein dieser Erfindung entsprechen, vom chemischen Konjugat. Anschließend werden die Proteine eluiert, welche nach In-Kontakt-Bringen des Aufschlusses mit dem chemischen Konjugat eine stabile Verbindung eingegangen sind. Dies kann beispielsweise durch Verwendung ansteigender Konzentrationen des Inhibitors geschehen,

welcher in dem chemischen Konjugat enthalten ist. Als Alternative kann ein anderer Inhibitor der intestinalen Cholesterinabsorption verwendet werden. Die eluierten Proteine können einer weiteren Reinigung beispielsweise mittels anderer chromatographischer Techniken wie Ionenaustauschchromatographie, Polyacrylamidelektrophorese, Gelchromatographie, Hochdruckflüssigkeitschromatographie unterworfen werden.

In einem Verfähren zur Gewinnung eines Proteins, wie vorstehend beschrieben, kann das chemische Konjugat ein Polymer, gegebenenfalls einen Linker, sowie einen inhibitor der Cholesterinabsorption aus der Saponln- oder 2-Azetidinon-Reihe insbesondere eine Verbindung der Formel I, II der III enthalten.

Die Beschreibung der Darstellung eines chemischen Konjugats mittels chemischer Synthese erfolgt In den Beispielen.

Die Erfindung beschreibt eine Verbindung der Formel I.

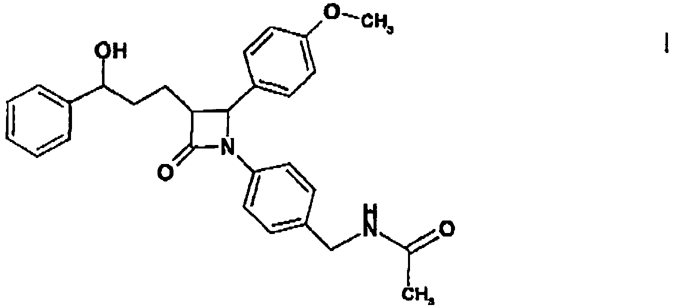

Die Erfindung beschereibt weiterhin eine Verbindung der Formel II.

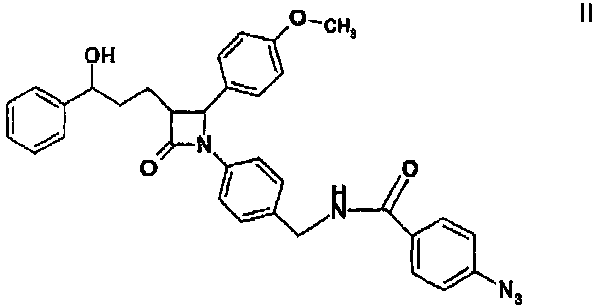

Die Erfindung beschreibt auch eine Verbindung der Formel III.

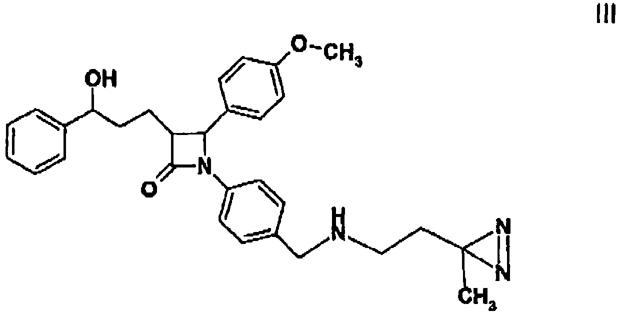

Eine Verbindung der Formel I, II oder III kann zum Nachweis eines Proteins das gemäß dem Verfahren dieser Erfindung gewonner wird (Photolabel) verwendet werden. Eine Verbindung der Formel I, II oder III eignet sich auch als Inhibitor eines cholesterinabsorbierenden Proteins.

Die Erfindung beschreibt auch die Verbindungen 4-[3-(3-Brom-3-phenyl-propyl)-2-(4-methoxy phenyl)-4-oxo-azetidin-1-yl]-benzonitril, 4-[3-(3-Hydroxy-3-phenylpropyl)-2-(4-methoxy-phenyl)-4-oxo-azetidin-1-yl]- benzonitril oder 1-(4-Aminomethylphenyl)-3-(3-hydroxy-3-phenyl-propyl)-4-(4-methoxy-phenyl)-azetindin-2-on. Diese soeben genannten Verbindungen können zur Herstellung einer Verbindung der Formel I, II oder III verwendet wenden.

### Beispiele:

Es wurden Inhibitoren der Intestinalen Cholesterinabsorption synthetisiert (Verbindungen der Formel I, II oder III). Diese Verbindungen enthalten photolabile Gruppen, die mittels UV-Licht mit anderen Molekülen insbesondere Proteinen quervernetzt werden können, und sind radioaktiv markiert mit einer spezifischen Aktivität von größer 1 Ci/mmol. Diese Verbindungen wurden verwendet, um ein Protein der intestinalen Cholesterinabsorption zu identifizieren.

### Beispiel 1:

### Photoaffinität und Bindungsstudien:

Vesikel aus dem Bürstensaumgewebe des Dünndarms von Kaninchen wurden mittels dem Fachmann bekannten Methoden isoliert (Kramer et al. J. Biol. Chem. 268, 18035 - 18046 (1993)). Die Photoaffinitätsmarkierung unter Verwendung einer radioaktiv markierten Verbindung der Formel I, II der III wurde in einem photochemischen Reaktor des Typs Rayonet RPR-100 (erhältlich von der Firma. The Southern Ultraviolet Company, Hamden, CT") ausgeführt. Die Bürstensaumvesikel davon (100 bis 200 µg Protein) wurden mit einer der Verbindungen für 5 Min. bei 20 °C im Dunkeln in einem Volumen von 200 µl in 10 mM Tris/Hepes-Puffer (pH 7,4), 100 mM NaCl. 100 mM Mannitol inkubiert. Anstelle der Bürstensaumvesikel können auch Organellen insbesondere Membranen aus diesen verwendet werden. Für diese gilt das im folgenden ausgeführte entsprechend. Nach der Inkubation im Dunkeln wurde mit UV-Licht von 254 nm für 20 Sekunden bzw. 60 Sekunden bestrahlt. Danach wurden die Bürstensaumvesikel zweimal mit dem genannten Puffer gewaschen. Die Proteine wurden mittels üblicher Techniken wie beispielsweise Versetzen mit Ethanol, Zugabe eines Salzes oder Detergens, Erhitzen, wiederholtes Einfrieren und Wiederauftauen oder einer anderen geeigneten und dem Fachmann bekannten Methode gefällt und durch SDScrylamidelektrophorese aufgetrennt. Die radioaktiv markierten Proteine konnten durch LSC oder Fluorographie nachgewiesen werden. Die Affinität der markierten Proteine aus dem Bürstensaumgewebe für die Verbindungen lag in einem Bereich von 1 bis 10 nM.

Identifizerung eines intestinalen Proteins, welches Cholesterin absorbiert.

Aus Bürstensaummembranen des Darms von Kaninchen wurden Vesikel präpariert. Diese wurden mittels einer radioaktiv markierten Verbindung der Formel I, II oder III markiert. Die Proteine wurden gefällt und auf einem SDS-Polyacrylamidgel aufgetrennt.

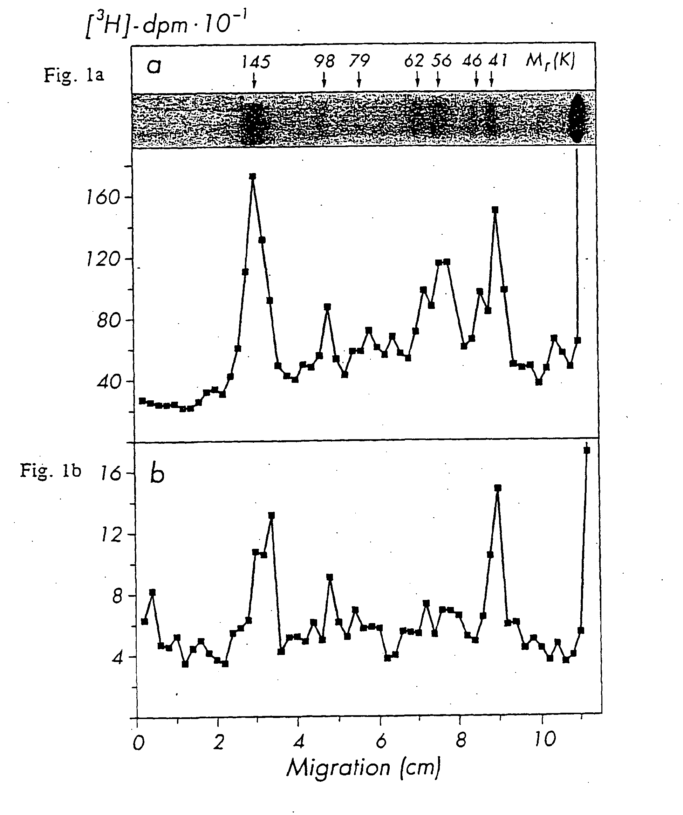

Bei Verwendung der Verbindung der Formel I konnten Proteine mit einem Molekulargewicht von 145 kDa, 97 kDa, 72 kDa, 63 kDa, 58 kDa, 41 kDa 32 kDa, und 25 kDa markiert werden (Fig. 1a). Mit der Verbindung der Formel II konnten Verbindungen mit einem Molekulargewicht von 145 kDa, 97 kDa, 87 kDa, und 41 kDa markiert werden. Den stärksten Einbau zeigte das Protein mit dem Molekulargewicht von 145 kDa. Durch Solubilisierungsexperimente mit nichtionischen oder zwitterionischen Detergentien und Natrium-Carbonat konnte für die Proteine mit dem Molekulargewicht 145 kDa, 97 kDa, 58 kDa und 32 kDa gezeigt werden, daß diese integrale Membranproteine sind. Die Angabe der Molekulargewichte der Proteine erfolgt vorbehaltlich eines gewissen Unschärfebereichs, welche bedingt ist durch die verwendete Methodik der SDS-Polyacrylamidgelelektrophorese., aber auch von anderen entsprechenden Methoden bekannt ist. Die Schwankungen der Molekulargewichte bewegen sich in einem Bereich von bis zu +/- 10 %. Die angegebenen Werte stellen die Mittelwerte aus mehreren Versuchen dar. Im Falle des Proteins mit dem angegebenen Molekulargewicht von 145 kDa ergab sich aus den Bestimmungen des Molekulargewichts von 10 unabhängig voneinander durchgeführten Versuchen mit Hilfe der SD8-Polyacrylamidgelelektrophorese ein Mittelwert von 145,3 kDa mit einer Standardabweichung von +/- 7,55 kDa.

Weitere Photomarkierungsstudien wurden mit bekannten Inhibitoren der Intestinalen Cholesterinabsorption durchgeführt Dazu wurden Derivate von S3302 hergestellt. Die Verbindung S3302 ist ein Razemat des Cholesterininhibitors SCH 48461. Diese Verbindung inhibiert sehr effektiv die intestinale Cholesterinabsorption in Kaninchen, Harnstern, Hunden, Rhesusaffen und dem Menschen (Davis et al., Atherosclerosis 109, 162 -163 (1994); Bergman et al.; XII International Symposium on Drugs affecting Lipid Metabolism; conference reports (1995)). Die Verbindung SCH 58235 (Literatur eben genannt) zeigt in Harnstern im direkten Vergleich eine 50 fach gesteigerte Wirkung. Dieser Effekt wird durch Einführung einer zusätzlichen (3S)-Hydroxy-Gruppe in Position R2 erklärt. Es konnte ein analoges Wasserstoff-Derivat von SCH 48461 hergestellt werden (= S6503), welches ähnliche Wirksamkeit im in vivo Versuch wie SCH 58235 zeigte. 56503 bewirkt in Analogie zu seiner starken in vivo Wirkung der Cholesterinresorption im Vergleich zu S3302 eine stärkere Hemmung der Markierung des Proteins mit dem Molekulargewicht von 145 kDa. Keiner der verwendeten Inhibitoren der Cholesterinabsorption zeigte in den in vivo Versuchen nennenswerten Einfluß auf Gailensalze, Glukose, Oligopeptide, Alanin, Fettsäuren oder den Na⁺/Gallensalz-Transportweg.

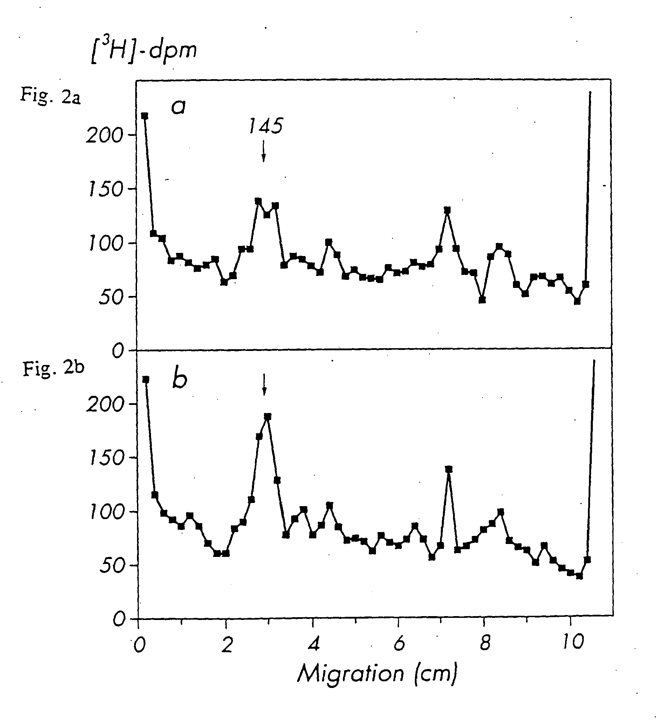

Photoaffinitätsstudien mit Bürstensaummembranen isoliert aus Kaninchen, denen für 10 Tage eine Diät mit hohen Cholesterinmengen verabreicht worden war, zeigten eine deutlich stärkere Markierung des Proteins mit denn Molekulargewicht von 145 kDa (Fig. 2).

Aus dem vorstehend ausgeführten ergibt sich, daß ein Protein dargestellt werden konnte, welches eine intestinales Cholesterinabsorption bewirkt. Damit konnte die Aufgabe dieser Erfindung gelöst werden.

### Beispiel 2:

Bereitstellung eines chemischen Konjugats aus einem Polymer und einer Verbindung, welche als Inhibitor der intestinalen Cholesterinabsorption wirkt Ein Aminoalkylderivat einer Verbindung, die als Inhibitor der intestinalen Cholesterinabsorption wirkt (5-100 µmoL), insbesondere eine Verbindung der Formel I, II oder III, wird In 1 - 5 ml Puffer / 0_{.}1 M NaHCO₃ /0,5 M NaCl (pH 8.3) gelöst. Polymere Trägermaterialien, die aktivierte Carboxylgruppen mit oder ohne Spacer enthalten, wie beispielsweise eine Hi-Trap Säule mit einer N-Hydroxysuccinimidyl-aktivierten Hexylcarbonsäure als Spacer (Pharmacia Biotechnology) werden entsprechend den Vorschriften des Herstellers zur Konjugation vorbereitet. Die aktivierte Matrix wird 0,5 - 5h mit obiger Lösung eines Aminoalkylderivats eines Cholesterinabsorptionshemmers inkubiert. Anschließend werden nicht umgesetzte aktivierte Carboxylgruppen durch mehrfaches sequentielles Spülen mit jeweils 5-15 ml folgender Puffer neutralisiert:
0,5 M Ethanolamin / 0,5 M NaCl (pH 8,3)
0,1 M Natriumacetat / 0.5 M NaCI (pH 4,0)

Anschließend wird die Konjugatsäule durch Spülen mit 10 mM Natriumphosphat-puffer (pH 7.4) /1% n-Octylglucosid für die Affinitätschromatographie vorbereitet.

Anreicherung von Bindeproteinen für Cholesterinabsorptionsinhibitoren durch Affinitätschromatographie

Biologische Membranen aus Zellen, welche Cholesterintransportproteine enthalten (2-20 mg Protein), wie beispielsweise Bürstensaummembranen von Dannzellen werden bei einer Proteinkonzentration von 0,5 - 2,5 mg/ml in 10 mM Natriumphosphatpuffer (pH 7,4) /1% n-Oktylglucosid 1 h bei 4 °C solubilisiert. Nach Abtrennung von verbleibendem partikulären Material durch Zentrifugation wird die klare überstehende Lösung, welche die solubilisierten Membranproteine enthält, auf die oben beschriebene Konjugatsäule aufgetragen und die Elution von Protein mittels UV-Absorption verfolgt. Nach Auswaschen nicht retardierter Proteine werden die an der Säule haftenden spezifischen Bindeproteine für Cholesterinabsorptionshemmer durch Aufbringen von Lösungen, welche Cholesterinabsorptionsinhibitoren in Konzentrationen von 1-10 mM enthalten oder die 1-5% an nichtionischen Detergentien wie Triton X-100 enthalten, eluiert.

Die Bindeproteine für Cholesterinabsorptionsinhibitoren werden anschließend in diesen Eluaten durch elektrophoretische Auftrennung separiert und ihre molekulare Größe bestimmt. Durch Ausschneiden der entsprechenden Proteinbanden kann mit dem Fachmann geläufigen Methoden durch Sequenzierung ihre Aminosäuresequenz bestimmt werden oder es können durch subkutane Deposition der Gelstücke, welche die aufgetrennten Proteine enthalten, in verschiedenen Tierspezies Antikörper gegen die einzelnen Proteine erzeugt werden.

### Beispiel 3:

Im folgenden wird die Herstellung einer Verbindung der Formel I, II oder III sowie eines radioaktiv markierten Derivats einer Verbindung der Formel I, II oder III beschrieben:

### 4-[3-(3-Brom-3-phenyl-propyl)-2-(4-methoxy-phenyl)-4-oxo-azetidin-1-yl]- benzonitril

16.0 g 4-[2-(4-Methoxy-phenyl)-4-oxo-3-(3-phenyl-propyl)-azetidin-1-yl]-benzonitril werden in 180 ml Tetrachlorkohlenstoff gelöst und mit 9.4 g N-Bromsuccinimid und 940 mg Benzoylperoxid versetzt. Man erwärmt unter Rühren zum Rückfluss und kontrolliert durch Dünnschichtchromatographie. Nach ca. 30-50 min ist die Umsetzung beendet. Die Lösung wird bei Raumtemperatur mit Ethylacetat versetzt, dann mit gesättigter Natriumbisufitlösung und Wasser gewaschen, über Natriumsulfat getrocknet und im Vakuum eingeengt. Der Rückstand wird über Kieselgel mit Ethylacetat/Heptan=1:2 als Laufmittel gereinigt. Man erhält das Bromid als zähes Öl.
MS(FAB): 477 (M1+H⁺),
475 (M2+H⁺) [C₂₆H₂₃BrN₂O₂ M=475].

### 4-[3-(3-Hydroxy-3-phenyl-propyl)-2-(4-methoxy-phenyl)-4-oxo-azetidin-1-yl]- benzonitril

17 g 4-[3-(3-Brom-3-phenyl-propyl)-2-(4-methoxy-phenyl)-4-oxo-azetidin-1-yl]-benzonitril werden in 500 ml Dioxan gelöst, mit 34 ml -40 proz. Tetrabutylammoniumtrifluoracetat-Lösung versetzt und 24 h zum Rückfluss erhitzt. Die Mischung wird auf ca. die Hälfte eingeengt, mit Wasser und Methyl-tert.-butyl-ether versetzt und in die Phasen getrennt. Die organische Phase wird eingeengt und der Rückstand mit 500 ml Ethanol und 200 ml konzentriertem Ammoniak 1 h bei Raumtemp. gerührt. Danach wird erneut im Vakuum eingeengt und das Rohprodukt an Kieselgel mit Dichlormethan/Methanol = 30:1 als Laufmittel gereinigt. Man erhält den Alkohol als Öl.
MS (ESI): 413 (M+H⁺), 395 (M+H⁺-H₂O) [C₂₆H₂₄N₂O₃ M=412].

### 1-(4-Aminomethyl-phenyl)-3-(3-hydroxy-3-phenyl-propyl)-4-(4-methoxy-phenyl)-azetidin-2- on

7g 4-[3-(3-Hydroxy-3-phenyl-propyl)-2-(4-methoxy-phenyl)-4-oxo-azetidin-1-yl]-benzonitril werden in 200 ml Ethanol gelöst und nach Zugabe von 15 ml konzentriertem Ammoniak mit 7 g 50% wässrigem Raney-Nickel bei 50 bar Wasserstoff hydriert. Das nach Absaugen des Katalysators und Entfernen des Lösungsmittels im Vakuum erhaltene Rohprodukt wird an Kieselgel mit Dichlormethan/Methanol/Ammoniak = 200:10:1 gereinigt. Man erhält das Amin. MS (FAB, + LiCl): 423 (M+Li⁺)
[C₂₆H₂₈N₂O₃ M=416].

### 4-Azido-N-{4-[3-(3-hydroxy-3-phenyl-propyl)-2-(4-methoxy-phenyl)-4-oxo-azetidin-1-yl]-benzyl}-benzamid

140 mg 1-(4-Aminomethyl-phenyl)-3-(3-hydroxy-3-phenyl-propyl)-4-(4-methoxyphenyl)-azetidin-2- on werden in 5 ml Dioxan gelöst und mit 0.1 ml Tributylamin versetzt. Dazu gibt man unter Ausschluss grellen Lichts 88 mg 4-Azido-benzoesäuresuccinimid-ester und rührt ca. 1 h bei Raumtemperatur nach. Das nach dem Einengen verbleibende Rohprodukt wird an Kieselgel mit Ethylacetat/Heptan = 1:1 als Laufmittel gereinigt. MS (FAB): 562 (M+H⁺), 544 (M+H⁺-H₂O) [C₃₃H₃₁N₅O₄ M=561].

Analog in kleinem Maßstab und hoher Verdünnung erhält man unter Verwendung von Tritium- oder ¹⁴C-markiertem Azidobenzoesäure-Aktivester (Fa. Amersham) radiomarkiertes Benzamid, das im Dünnschichtchromatogramm mit unmarkiertem 4-Azido-N-{4-[3-(3-hydroxy-3-phenyl-propyl)-2-(4-methoxy-phenyl)-4-oxo-azetidin-1-yl]-benzyl}-benzamid coeluiert.

### N-{4-[3-(3-Hydroxy-3-phenyl-propyl)-2-(4-methoxy-phenyl)-4-oxo-azetidin-1-yl]-benzyl}-acetamid

100 mg 1-(4-Aminomethyl-phenyl)-3-(3-hydroxy-3-phenyl-propyl)-4-(4-methoxyphenyl)-azetidin-2- on werden in 20 ml Methanol gelöst und nach Zugabe von 0.3 ml Pyridin mit 0.3 ml Acetanhydrid versetzt. Nach 30 min bei Raumtemperatur ist die Reaktion beendet, man engt im Vakuum ein und reinigt an Kieselgel mit Ethylacetat als Laufmittel. MS (ESI): 459(M+H⁺), 441 (M+H⁺-H₂O) [C₂₈H₃₀N₂O₄ M=458].

Analog in kleinem Maßstab und hoher Verdünnung erhält man unter Verwendung von Tritium- oder ¹⁴C-markiertem Acetanhydrid radiomarkiertes Acetamid, das mit unmarkiertem N-{4-[3-(3-Hydroxy-3-phenyl-propyl)-2-(4-methoxy-phenyl)-4-oxo-azetidin-1-yl]-benzyl}-acetamid im Dünnschichtchromatogramm coeluiert.

### 3-(3-Hydroxy-3-phenyl-propyl)-4-(4-methoxy-phenyl)-1-(4-{[2-(3-methyl-3H-diazirin-3-yl)-ethylamino]-methyl}-phenyl)-azetidin-2-on

100 mg 1-(4-Aminomethyl-phenyl)-3-(3-hydroxy-3-phenyl-propyl)-4-(4-methoxyphenyl)-azetidin-2- on werden in 5 ml Dimethylformamid gelöst und mit Toluol-4-sulfonsäure-2-(3-methyl-3H-diazirin-3-yl)-ethyl ester unter Vermeidung grellen Lichts 6 h bei 60-70°C gerührt. Man versetzt mit Wasser, extrahiert mit Ethylacetat und entfernt das Lösungsmittel im Vakuum. Anschließend wird zweimal mit trockenem Toluol versetzt und im Vakuum eingeengt. Das Rohprodukt wird an Kieselgel mit Dichlormethan/ Methanol = 20:1 als Laufmittel gereinigt.
MS (ESI): 499 (M+H⁺) [C₃₀H₃₄N₄O₃ M=498].

Durch Verwendung von Tritium- oder ¹⁴C-markiertem Diazirin erhält man auf gleichem Weg radiomarkiertes Azetidinon, das im Dünnschichtchromatogramm mit unmarkiertem 3-(3-Hydroxy-3-phenyl-propyl)-4-(4-methoxy-phenyl)-1-(4-{[2-(3-methyl-3H-diazirin-3-yl)-ethylamino]-methyl}-phenyl)-azetidin-2-on coeluiert.

### Erklärung der Figuren:

Fig 1a, Fig. 1b:
   Markierung von Proteinen der Bürstensaummembran von Kaninchen mit radioaktiv markierten Verbindungen der Formel I oder II. Die Proteine wurden gefällt und auf einem SDS-Polyacrylamidgel aufgetrennt. Die Menge an Radioaktivität wurde mittels quantitativer Fluorographie bestimmt. Auf der horizontalen Achse der Diagramme ist der Abstand vom Auftrag in cm, auf der vertikalen Achse das Zählergebnis in ³H-dpm aufgetragen. Die Fig. 1a zeigt im oberen Teil die Verteilung der Radioaktivität nach Markierung mit radioaktiv markierter Verbindung der Formel II und fluorographischem Nachweis.
   In Fig. 1 a wurde mit einer Verbindung der Formel I und in Fig. 1b mit einer Verbindung der Formel II markiert.
   Spezifisch markierte Proteine sind an den Peaks erkennbar.
Fig 2a, 2b:
   Kaninchen wurden 10 Tage mit einer Diät gefüttert, die hohe Konzentrationen an Cholesterin enthielt. Danach wurden Vesikel der Bürstensaummembranen dieser Tiere präpariert, anschließend wie beschrieben durch eine Verbindung der Formel I oder II behandelt, danach wurden die Proteine gefällt und auf einem SDS-Polyacrylamidgel aufgetrennt. Die Menge an Radioaktivität wurde mittels quantitativer Fluorographie bestimmt. Auf der horizontalen Achse der Diagramme ist der Abstand vom Auftrag in cm, auf der vertikalen Achse das Zählergebnis in ³H-dpm aufgetragen.
   In Fig. 2a wurde mit einer Verbindung der Formel I und in Fig. 2b mit einer Verbindung der Formel II markiert.
   Man erhielt eine spezifische Markierung eines Proteins mit einem Molekulargewicht von 145 kDa.

### Abkürzungen:

- h: Stunde(n)
- LSC: Liquid Szintillation Counting
- MS (FAB): Massenspektrum (Fast Atom Bombardment)
- MS (ESI): Massenspektrum (Electrospray Ionisierung)
- M: Masse

## Patentansprüche

1. Verfahren zur Isolierung eines Cholesterin absorbierenden Proteins aus dem Darmgewebe eines Säuger-Organismus, wobei das Verfahren die folgenden Verfahrensschritte umfasst:
a) Bereitstellung von Zellen aus dem Bürstensaum des Darmgewebes oder Teilen von diesen Darmzellen eines Säuger-Organismus,
b) Bereitstellung einer radioaktiv markierten Verbindung, welche als Inhibitor der intestinalen Cholesterinabsorption wirkt, und eine photolabile Gruppe enthält,
c) In-Kontakt-Bringen der Darmzellen oder Teilen von diesen Darmzellen aus a) mit einer Verbindung aus b),
d) Bestrahlung des Ansatzes gemäss c) mit UV-Licht,
e) Aufschluss der Zellen nach Bestrahlung gemäss d),
f) Auftrennung der Bestandteile des Aufschlusses der Zellen nach Aufschluss gemäss e),
g) Nachweis eines Proteins nach Auftrennung gemäss f), welches eine Verbindung gemäss b) kovalent gebunden enthält,
dadurch charakterisiert, dass das Protein eine Größe von 145 kDa +/- 7,55 kDa bei der SDS-Polyacrylamid-Gelelektrophorese hat, dass das Protein ein integrales Membranprotein ist und dass das Protein in glykosyliertern Zustand vorliegt,
wobei die Verbindung gemäss Verfahrenschritt b) eine Verbindung mit folgender Formel I 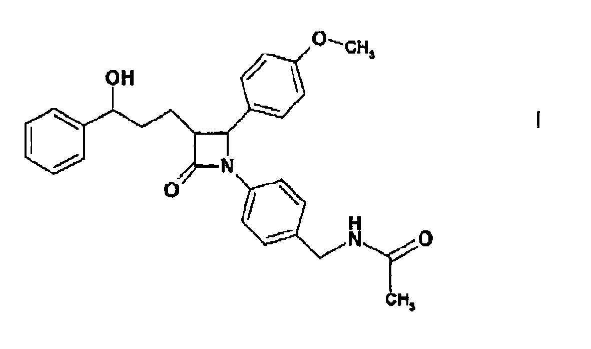 ist oder eine Verbindung mit folgender Formel II 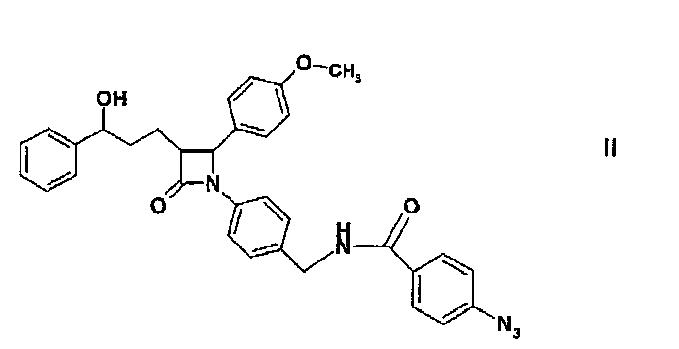 ist.

2. Verfahren nach Anspruch 1, wobei die Darmzellen gemäss Verfahrensschritt a) von Mensch, Affe, Ratte; Rind, Schwein, Maus, Kaninchen oder Hamster stammen.

3. Verfahren nach Anspruch 1 oder 2, wobei das Protein einen Inhibitor der intestinalen Cholesterinabsorption binden kann.

4. Verfahren nach Anspruch 3, wobei das Protein Azetidinone oder Saponine binden kann.

5. Verfahren nach einem oder mehreren der Ansprüche 1 bis 4, wobei das Protein Cholesterin binden kann.

## Claims

1. A method for isolating a cholesterol-absorbing protein from the intestinal tissue of a mammalian organism, where the method comprises the following process steps:
a) provision of cells from the brush border of the intestinal tissue or parts of these intestinal cells of a mammalian organism,
b) provision of a radiolabeled compound which acts as inhibitor of intestinal cholesterol absorption and contains a photolabile group,
c) contacting the intestinal cells or parts of these intestinal cells from a) with a compound from b)
d) irradiation of the mixture from c) with UV light,
e) disruption of the cells after irradiation as in d),
f) fractionation of the components of the disrupted cells after disruption as in e),
g) detection, after fractionation as in f), of a protein which contains a covalently bound compound from b),
**characterized in that** the protein has a size of 145 kDa +/- 7.55 kDa in SDS polyacrylamide gel electrophoresis, that the protein is an integral membrane protein and that the protein is present in a glycosylated state,
where the compound in process step b) is a compound having the following formula I 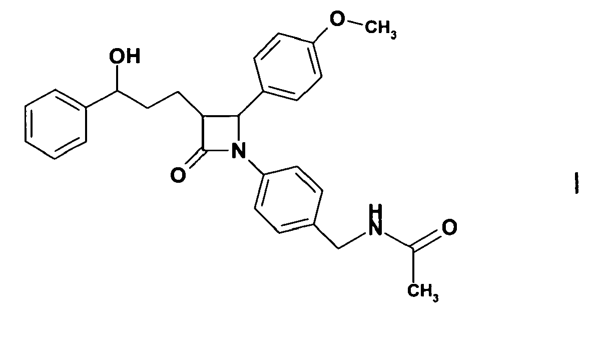 or is a compound having the following formula II

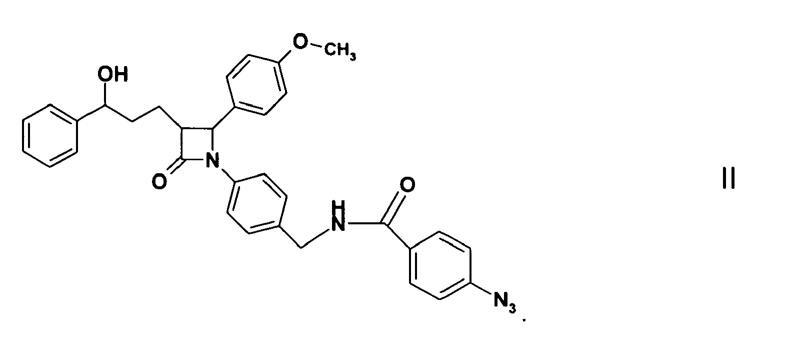

2. A method as claimed in claim 1, where the intestinal cells in process step a) originate from humans, monkeys, rats, cattle, pigs, mice, rabbits or hamsters.

3. A method as claimed in claim 1 or 2, where the protein is able to bind an inhibitor of intestinal cholesterol absorption.

4. A method as claimed in claim 3, where the protein is able to bind azetidinones or saponins.

5. A method as claimed in one or more of claims 1 to 4, where the protein is able to bind cholesterol.

## Revendications

1. Procédé pour isoler une protéine absorbant le cholestérol à partir du tissu intestinal d'un organisme de mammifère, le procédé comprenant les étapes suivantes :
a) mise à disposition de cellules provenant de la bordure en brosse du tissu intestinal ou de parties de ces cellules intestinales d'un organisme de mammifère,
b) mise à disposition d'un composé radiomarqué, qui agit en tant qu'inhibiteur de l'absorption intestinale du cholestérol, et contient un groupe photolabile,
c) mise en contact des cellules intestinales ou des parties de ces cellules intestinales de a) avec un composé de b),
d) irradiation de la masse réactionnelle selon c) avec une lumière UV,
e) désintégration des cellules après irradiation selon d),
f) fractionnement des constituants de la désintégration des cellules après la désintégration selon e),
g) détection d'une protéine après le fractionnement selon f), qui contient, lié d'une manière covalente, un composé selon b),
**caractérisé en ce que** la protéine a une taille de 145 kDa ± 7,55 kDa, déterminée par électrophorèse sur gel de polyacrylamide en présence de SDS; que la protéine est une protéine membranaire intégrale, et que la protéine se présente à l'état glycosylé,
le composé selon l'étape b) étant un composé ayant la formule I ci-après 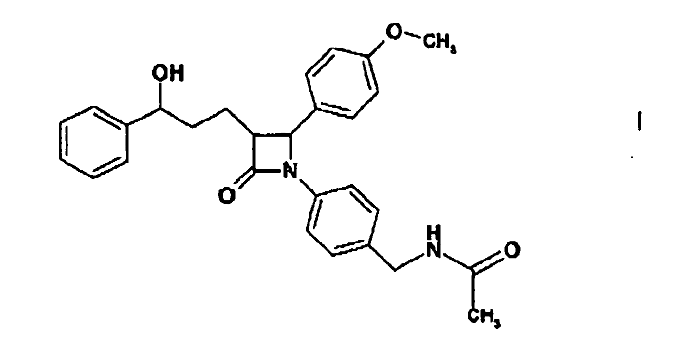 ou un composé ayant la formule II ci-après

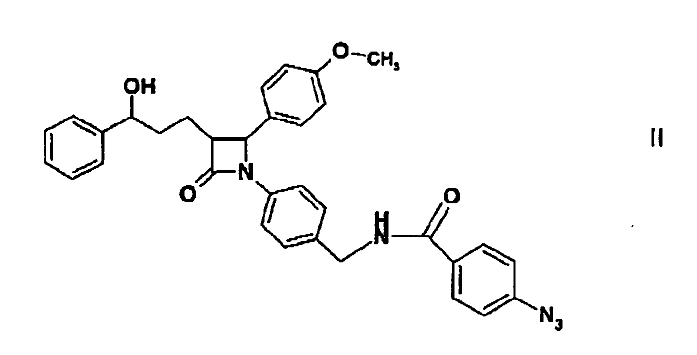

2. Procédé selon la revendication 1, dans lequel les cellules intestinales selon l'étape a) proviennent d'un humain, d'un singe, d'un rat, d'un bovin, d'un porc, d'une souris, d'un lapin ou d'un hamster.

3. Procédé selon la revendication 1 ou 2, dans lequel la protéine peut fixer un inhibiteur de l'absorption intestinale du cholestérol.

4. Procédé selon la revendication 3, dans lequel la protéine peut fixer des azétidinones ou des saponines.

5. Procédé selon l'une ou plusieurs des revendications 1 à 4, dans lequel la protéine peut fixer le cholestérol.
